# EUROPEAN PATENT SPECIFICATION

(11) **EP 1 901 753 B1**
(45) Date of publication and mention of the grant of the patent: **08.05.2013**
(21) Application number: 06744275.6
(22) Date of filing: 22.06.2006
(51) Int. Cl.: A61K 31/58, A61K 45/00, G01N 33/50, G01N 33/53, G01N 33/566, A61P 25/28

(54) **MULTIPLE SCLEROSIS THERAPY AND DIAGNOSIS**
BEHANDLUNG UND DIAGNOSE DER MULTIPLEN SKLEROSE
TRAITEMENT ET DIAGNOSTIC DE LA SCLEROSE EN PLAQUES

(30) Priority: 22.06.2005 GB 0512726
(43) Date of publication of application: 26.03.2008
(73) Proprietor: MS Therapeutics Limited, Crowthorne, Berkshire RG45 7AW (GB)
(72) Inventor: CHIBBER, Rakesh, 57 Waterloo Road London SE1 8WA (GB); HAGAN, Russell, London EC4M 7SB (GB)
(74) Representative: Kirkham, Nicholas Andrew
(86) International application number: PCT/GB2006/002301
(87) International publication number: WO 2006/136841

(56) References cited:
- WO-A-00/31109
- ORLACCHIO A ET AL: "Activity levels of a beta1,6 N-acetylglucosaminyltransferase in lymphomonocytes from multiple sclerosis patients" JOURNAL OF NEUROLOGICAL SCIENCES, ELSEVIER SCIENTIFIC PUBLISHING CO, AMSTERDAM, NL, vol. 151, no. 2, 22 October 1997 (1997-10-22), pages 177-183, XP002232475 ISSN: 0022-510X cited in the application

## Description

The present invention relates to treatments for and diagnosis of neuroinflammatory diseases and in particular multiple sclerosis (MS).

Multiple sclerosis is a disease with a significant inflammatory component. The enzyme Core 2 GlcNAc-T is involved in the synthesis of branched chain, O-linked oligosaccharides. Core 2 GlcNAc-T - EC 2.4.1.102 is also known as UDP-GlcNAc:Galß1,3GalNAc-R (GlcNAc to GalNAc) ß-1,6-N-acetylglucosaminyl transferase or Core 2 ß-1,6 N-acetylaminotransferase.

Although this enzyme has been implicated in inflammation (WO 0031109), Orlacchio A. et al (1997) J Neurol Sci. 22;151(2):177-83 discloses that the level of activity of Core 2 GlcNAc-T is reduced in lymphomonocytes from patients with both relapsing remitting and progressive MS.

The present inventors have now surprisingly determined that Core 2 GlcNAc-T activity is in fact significantly raised in leukocyte preparations containing peripheral blood mononuclear cells (PBMNC) and polymorphonuclear (PMN) leukocytes from sample patients with MS. As Core 2 based oligosaccharides are found *inter alia* as a component of the ligands of proteins that are thought to mediate aspects of cell adhesion during the inflammatory response this has implications for increased leukocyte infiltration of tissues in MS. As raised Core 2 GlcNAc-T contributes to increased adhesiveness of leukocytes, the present inventors have determined that lowering the activity of Core 2 GlcNAc-T should tend to normalise adhesiveness of leukocytes, reduce leukocyte extravasation and reduce neuro-inflammation and associated plaque in MS patients.

Accordingly in a first aspect of the invention is provided a method of treatment of Multiple Sclerosis in a subject comprising administering to a subject in need thereof, a therapeutically effective amount of a compound capable of reducing the activity of Core 2 GlcNAc-T. Preferably the compound will be used to reduce the activity of Core 2 GlcNAc-T to normal or approximately normal levels.

The activity of Core 2 GlcNAc-T can be reduced in a number of ways, for example by inhibiting the transcription of the Core 2 GlcNAc-T gene, by inhibiting the translation of the Core 2 GlcNAc-T mRNA, by inhibiting the post translational modification of the protein (e.g. by inhibiting the phosphorylation of the protein through protein kinase and thereby inhibiting its activation) or by inhibiting the enzyme activity.

Inhibitors of both Core 2 GlcNAc-T enzyme activity and of the activation of Core 2 GlcNAc-T by protein kinase C are known. Conveniently the level of Core 2 GlcNAc-T enzyme activity is reduced either by inhibiting the enzyme or inhibiting the phosphorylation of the protein.

Examples of Core 2 GlcNAc-T inhibitors suitable for use in the invention are: ßGal(1→3)α(6-deoxy)GalNAcα-Bn. (Hindsgaul et al (1991) J Biol Chem. 266(27):17858-62, Kuhns et al (1993) Glycoconjugate Journal 10, 381-394 ; the following compounds activated as described by Toki et al (1994) Biochem Biophys Res Commun. 198(2):417-23.: Galß1→3GalNAcα-pnp, Galß1→3GalNAcα-onp GalNAcα-pnp GalNAcß-pnp, GlcNAcß-pnp, Galß-pnp, GlcNAcß1→3GalNAcα-pnp, L-Fucα1→2Galß-pnp, GlcNAcα-pnp, Galß1→3 GlcNAcß-pn, Galß1→6GlcNAcß-pnp; steroidal glycosides described in applicants co pending WO05060977, eg. Trigoneoside IVa, Glycoside F, Compound 3 (3ß-26-(ß-D-glucopyranosyloxy)-22-hydroxyfurost-5, 25 (27)dien-3-yl O-6-deoxy-α-L-mannopyranosyl-(1→2)-O-[ß-D-glucopyranosyl-(1→4)]-ß-D-gluco-pyranoside), Pardarinoside C, Shatavarin I, Shatavarin IV, Deltonin, Balanitin VI, solasodine 3-O-α-L-rhamnopyranosyl-(1→2)-O-[ß-D-glucopyranosyl-(1→4)]-ß-D-glucopyranoside, solandine 3-O-α-L-rhamnopyranosyl-(1→2)-O-[ß-D-glucopyranosyl-(1→4)]-ß-D-glucopyranoside; and analogues of uridine diphosphate and uridine diphosphate-N-acetylglucosamine and peptides of the formula X-X¹-3⁰-X³-X⁴ as described in WO0185748.

Antibodies to Core 2 GlcNAc-T may also be used to reduce the activity of the enzyme and suitable examples are described in Li et al (1999) Glycoconjugate Journal 16, 555-562 (1999), US5684134, WO09043662).

Inhibitors of Protein Kinase-Cß2 (PKCß2) are known to inhibit Core 2 GlcNAc-T activation in diabetic complications, where Core 2 activity is known to be raised, (Chibber et al (2003) Diabetes. 52(6):1519-27-) and are known to inhibit leukocyte binding to epithelial cells *in vitro.* Examples of 3,4-di-indoyl-pyrrol-2,5-dione derivatives that inhibit PKCß are found in, for example WO9535294 and W09517182. A particular example of a PKCß2 inhibitor is Ruboxistaurin (LY333531 & LY379196)

In a second aspect of the invention is provided the use of compound capable of reducing Core 2 GlcNAc-T activity in the manufacture of a medicament for the treatment of Multiple Sclerosis. Examples of such compounds are as described above in the first aspect of the invention. For example such compounds are either inhibitors of Core 2 GlcNAc-T or inhibitors of PKCß (especially of PKCß2); preferably compounds are inhibitors of Core 2 GlcNAc-T.

In a third aspect of the invention is provided a pharmaceutical composition for the treatment of Multiple Sclerosis comprising a compound capable of lowering the activity of Core 2 GlcNAc-T and preferably also comprising a pharmaceutically acceptable carrier.

In a fourth aspect of the invention is provided a method of diagnosing Multiple Sclerosis in a subject comprising comparing the level of Core 2 GlcNAc-T activity associated with leukocytes of a subject with the level of Core 2 GlcNAc-T activity determined in healthy non afflicted individuals. A level of Core 2 GlcNAc-T higher than that of healthy non afflicted individuals being indicative that the subject is afflicted with MS.

The measurement of Core 2 GlcNAc-T activity is preferably carried out on isolated tissue samples, such as biopsy samples or blood samples. Conveniently the measurement will be carried out by assay of Core 2 GlcNAC-T from isolated blood cells and particularly on preparations containing leukocytes, preferably substantially free of red blood cells. One such suitable procedure using leukocytes isolated from blood samples is described in Chibber et al Diabetes 49, 1724-1730 (2000). Typically values of Core 2 GlcNAc-T activity associated with leukocytes of a subject will be compared to an established normal level for healthy non afflicted individuals.

The inventors have determined that the level of Core 2 GlcNAc-T activity in leukocyte preparations obtained from healthy individuals and assayed by the method of Chibber et al (2000) *id* or as detailed in Example 1 is between 40 and 1000 pmoles/hr/mg (oligosaccharide incorporated per mg protein) and typically between 50 and 500 pmoles/hr/mg of protein. values obtained for three groups of healthy control individuals were 249 ± 35 9 (n=25), 334 ± 86 (n=11) and 283 ± 37 (n=31) pmols/hr/mg.

Levels of Core 2 GlcNAc-T in individuals afflicted with MS have been noted to be in the region of at least 2 times, for example at least 4 times, at least 6 times and most typically at least 8 times the level of healthy non afflicted individuals when leukocytes from blood samples assayed according to the above methods.

In a fifth aspect of the invention is provided a method of determining the utility of a test substance as useful in the treatment of MS comprising determining the ability of the substance to inhibit the activity of Core 2 GlcNAc-T, particularly that activity associated with leukocytes.

Conveniently inhibition of Core 2 GlcNAc-T activity can be determined by comparing the level of Core 2 GlcNAc-T activity obtained in an assay in which a test substance is incorporated to the level of Core 2 GlcNAc-T activity in the assay with no test substance.

Conveniently inhibition of Core 2 GlcNAc-T enzyme activity can be determined by a method comprising (a) contacting source of active Core 2 GlcNAc-T enzyme with an acceptor and a sugar donor for a Core 2 GlcNAc-T in the presence and absence of the test substance; (b) measuring the amount of sugar donor transferred to the acceptor, and relating decreased transfer in presence of test substance as compared to that in its absence to Core 2 GIcNAc-T inhibitory activity. It is particularly preferred and convenient to measure such activity on Core 2 GlcNAc-T present in or derived from leukocytes, particularly of an MS patient.

Any source of Core 2 GlcNAc-T activity may be used, for example an enzyme produced by recombinant means such as those disclosed in WO04111196, US5658778 or a tissue or cell culture or a preparation exhibiting measurable Core 2 GlcNAc-T activity derivable therefrom, for example U937 cells or heart lysates as described in applicants co pending application PCT GB/2004/005398).

Examples of sugar donors and acceptors and the general conditions for assaying Core 2 GlcNAc-T activity are well known in the art e.g. *Chibber et al* (2000) *id* Hindsgaul *et al* (1991) *id,* Kuhns *et al* (1993) *id* Toki *et al* (1994) *id* and Orlacchio *et al* (1997) *id*. Such methods can be adapted for use in the fourth aspect of the invention by incorporation of test substances as described above. Further examples of assays according to the invention are given in WO 0031109 and in applicants copending application PCT GB/2004/005398. Conveniently the sugar donor is UDP-GIcNAc and the sugar acceptor is ßGal(1-3)DαGalNAc-*p*-nitrophenol.

The term treating MS, as used herein, includes treating as prophylaxis and the treatment of existing disease. MS includes for example relapsing/remitting, secondary progressive, progressive relapsing and primary progressive forms of the condition. Other forms include benign, malignant, chronic/progressive and transitional/progressive MS.

Medicaments comprising compounds that reduce the activity of Core 2 GlcNAc-T, unless in raw foodstuff form, will preferably be provided sterile and or pyrogen free. Particularly aqueous medicaments such as aqueous solutions or suspensions and especially those for use in parenteral applications, will be made up in sterile and pyrogen free water. Medicaments of the second embodiment comprising the compounds of the invention can be administered by oral or parenteral routes, including intravenous, intramuscular, intraperitoneal, subcutaneous, transdermal, airway (aerosol), rectal, vaginal and topical (including buccal and sublingual) administration. For oral administration, the compounds of the invention will generally be provided in the form of tablets or capsules, as a powder or granules, or as an aqueous solution or suspension.

Tablets for oral use may include the active ingredients mixed with pharmaceutically acceptable excipients such as inert diluents, disintegrating agents, binding agents, lubricating agents, sweetening agents, flavouring agents, colouring agents and preservatives. Examples of suitable inert diluents include sodium and calcium carbonate, sodium and calcium phosphate, and lactose, while corn starch and alginic acid are examples of suitable disintegrating agents. Binding agents include, for example starch and gelatine, while the lubricating agent, if present, may for example, be magnesium stearate, stearic acid or talc. If desired, the tablets may be coated with an enteric coating material, such as glyceryl mono stearate or glyceryl distearate, to delay absorption in the gastrointestinal tract. Capsules for oral use include hard gelatine capsules in which the active ingredient is mixed with a solid diluent, and soft gelatine capsules wherein the active ingredients is mixed with water or an oil such as peanut oil, liquid paraffin or olive oil

Formulations for rectal administration may for example be presented as a suppository with a suitable base comprising, for example, cocoa butter or a salicylate.

Formulations suitable for vaginal administration may for example be presented as pessaries, tampons, creams, gels, pastes, foams or spray formulations containing in addition to the active ingredient such carriers as are known in the art to be appropriate.

For intramuscular, intraperitoneal, subcutaneous and intravenous use, the compounds of the invention will generally be provided in sterile solutions or suspensions, buffered to an appropriate pH and isotonicity. For example suitable aqueous vehicles include Ringer's solution and isotonic sodium chloride. Aqueous suspensions according to the invention may include suspending agents such as cellulose derivatives, sodium alginate, polyvinylpyrrolidone and gum tragacanth, and a wetting agent such as lecithin. Liposome formulations may also be used. Suitable preservatives include ethyl and n-propyl p-hydroxybenzoate.

The Core 2 GIcNAc-T inhibitors of the invention may also be incorporated to a food or beverage product.

In general a suitable dose of Core 2 GlcNAc-T inhibitor will be in the range of 10 ng to 50 mg per kilogram body weight of the recipient per day, preferably in the range of 100ng to 10mg, more preferably µg to 1.0 mg/kg/d. The desired dose is preferably presented once daily. These sub-doses may be administered in unit dosage forms, for example, containing 10 µg to 1500 mg, preferably 100µg to 1000 mg, and most preferably 1mg to 700 mg of active ingredient per unit dosage form.

The present invention will now be described further by reference to the following non-limiting Examples, Schemes and Figures. Further embodiments falling within the scope of the invention will occur to those skilled in the art in the light of these

### FIGURES

Figure 1 is a graph illustrating the levels of Core 2 GlcNAc-T activity in leukocytes from healthy control individuals and subjects with newly diagnosed MS.
Figure 2 is a graph illustrating the data of figure 1 as a scatter plot.

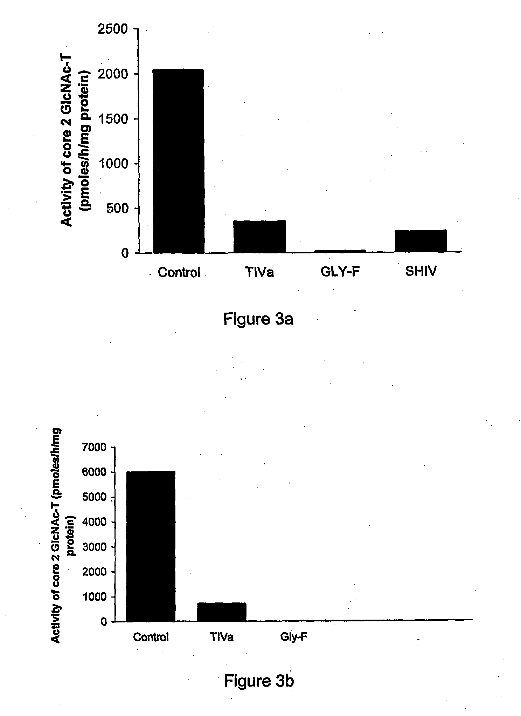

Figures 3a and 3b are graphs illustrating the effect of purified trigoneoside IVa, glycoside F, and shatavarin IV on Core 2 GlcNAc-T activity in cell free (Figure 3a) and cell based (figure 3b) assays. Test compounds were used at a final concentration of 20ng/ml. TIVa = Trigoneoside IVa, Gly-F = Glycoside F, SHIV = Shatavarin IV.

### EXAMPLES

### Example 1. Determination of Core 2 GlcNAc-T activity in leukocytes isolated from patients newly diagnosed with MS.

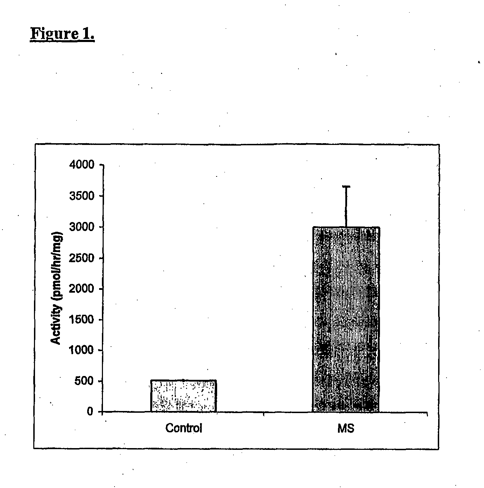

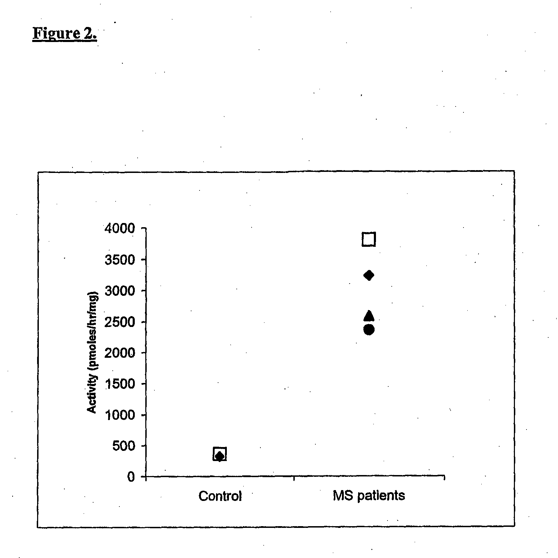

Blood samples were taken from 4 patients newly diagnosed with active MS and 2 age matched healthy control subjects and placed in heparinised tubes. The blood sample was layered onto an equal volume of Histo-Paque 1077^{™} (Sigma, Poole, Dorset, UK). and centrifuged at 400g for 30 mins. The Buffy coat (containing peripheral blood mononuclear cells (PBMNC) and polymorphonuclear (PMN) leukocytes) was washed in phosphate buffered saline. Isolated leukocytes were frozen and lysed in 0.9% NaCl 0.4% Triton-X100 1mM PMSF and the Core 2 GlcNAc-T assayed. The reaction was performed in 50 mmol/l 2(N-morpholino) 2(N-morpholino) ethanesulfonic acid pH 7.0; 1 mmol/l UDP GlcNAc, 0.5µCi UDP-6 [3H]-N-acetylglucosamine (16,000 dpm/nmol, NEN Life Science Products, Hounslow,U.K.); 0.1 mol/l GlcNAc; 1 mmol/l ßDgal (1-3)Dα-GalNAc-p-nitrophenol and 15 µl cell lysate (100-200 µg protein) for a final volume of 30 µl. After incubating the mixture for 1 h at 37°C, the reaction was terminated by adding 1ml of ice cold water and processed on a C18 Sep-Pak column (Waters-Millipore, Watford, U.K.). After washing the column with 20 ml water, the product was eluted with 5 ml methanol and radioactivity counted. Endogenous activity of core 2 transferase was measured in the absence of the added acceptor. The results are shown in figures 1 and 2.

### 2. Inhibition of Core 2 GlcNAc-T activity by inhibitors of Core 2 GlcNAc-T

### 2a Cell based assay

Human leukocytes (U937 cells) were exposed to human recombinant TNF-alpha (8pg/ml) in the presence and absence of test compounds After 24h incubation, the activity of Core 2 GlcNAc- T was measured as above.

### 2b Cell free assay

In cell free assays of Core 2 GlcNAc-T Heart lysates from either from TNF-alpha over expressing transgenic mice (female, B6.SJL-Tg (TNF) supplied by Taconic-M+B, Bomholtveg 10, 8680 Ry, Denmark) or from BB rats (Festing M.F.W. (Ed.). Inbred strains in biomedical research. The Macmillan Press Ltd, London (1979). ISBN 0-333-23809-5) were exposed to various concentrations of test compound for 1h at 37°C. Activity of Core 2 GlcNAc-T was measured as above.

Trigoneoside IVa and Glycoside F purified from fenugreek seeds (Yoshikawa et al 1998 Heterocycles 47, 397-405 ) and Shatavarin IV (Ravikumar et al 1987 Indian J. Chem. 26B, 1012-1017, Joshi and Dev 1988 Indian J. Chem. 27B, 12-16) were tested as inhibitors in the above assays.

**Table 1. Approximate IC₅₀ values (nM) for Core 2 GlcNAc-T inhibitors**

| Compound | Cell free assay | Cell based assay |
|---|---|---|
| Trigoneoside IVa | 0.9 * | 0.75 |
| Glycoside F | 5 ** | a |
| Shatavarin IV | b | c |

| | | |
|---|---|---|
| * Cell free assays were carried out on heart lysates of TNF-α mice ** Cell free assays were carried out on heart lysates ofBB rats a 100% inhibition at 22nM in BB rat heart lysate b 89% inhibition at 22nM in BB rat heart lysate c no activity detected at 22.5 nM | | |

## Claims

1. A compound capable of reducing the activity of Core 2 GlcNAc-T for use in the treatment of multiple sclerosis

2. A compound for use according to Claim 1 wherein the compound capable of reducing the activity of Core 2 GlcNAc-T is an inhibitor of Core 2 GlcNAc-T enzyme activity.

3. A compound for use according to either of Claims 1 or 2 in which the inhibitor of Core 2 GlcNAc-T activity is selected from the group consisting of steroidal glycosides, analogues of Uridine Diphosphate-N-Acetylglucosamine, analogues of Uridine Diphosphate, ßGal(1→3)α(6-deoxy)GalNAcα-Bn and those compounds obtainable by UV activation of a compounds selected from the group consisting of Galß1→3GalNAcα-pnp, Gaß1→3GalNAcα-onp, GalNAcα-pnp, GalNAcß-pnp,
GlcNAcß-pnp, Galß-pnp, GlcNAcß1→3GalNAcα-pnp, L-Fucα1→2Galß-pnp, GlcNAcα-pnp, Galß1→6GlcNAcß-pnp and Galß1→3GlcNAcß-pnp.

4. A compound for use according to either one of Claims 2 or 3 in which the inhibitor of Core 2 GlcNAc-T activity is a steroidal glycoside.

5. A compound for use according to any one of Claims 2 to 4 in which the inhibitor of Core 2 GlcNAc-T activity is selected from the group consisting of Trigoneoside IVa, Glycoside F, Compound 3, Pardarinoside C, Shatavarin I, Shatavarin IV, Deltonin, Balanitin VI, solasodine 3-O-α-L-rhamnopyranosyl-(1→2)-O-[ß-D-glucopyranosyl-(1→4)]-ß-D-glucopyranoside, Solanidine 3-O-α-L-rhamnopyranosyl-(1→2)-O-[ß-D-glucopyranosyl-(1→4)]-ß-D-glucopyranoside.

6. A compound for use according to any one of Claims 2 to 5 in which the inhibitor of Core 2 GlcNAc-T activity has been incorporated into a food or beverage product

7. A compound for use according to Claim 1 wherein the compound capable of reducing the activity of Core 2 GlcNAc-T is an inhibitor of protein kinase Cß.

8. A compounds for use according to Claim 7 wherein the compound capable of reducing the activity of Core 2 GlcNAc-T is an inhibitor of protein kinase Cß2.

9. A compounds for use according to Claim 8 wherein the compound is selected from the group consisting of 3,4-di-indoyl-pyrrol-2,5-dione derivatives.

10. A compounds for use according to Claim 8 wherein the compound is Raboxistaurin.

11. A compound for use according to any one of Claims 1 to 10 wherein Multiple Sclerosis is selected from relapsing/remitting, secondary progressive, progressive relapsing, primary progressive benign, malignant, chronic/progressive and transitional/progressive Multiple Sclerosis.

12. A method of diagnosing Multiple Sclerosis in a subject comprising comparing the level of Core 2 GlcNAc-T activity associated with samples isolated from the subject with the level of Core 2 GlcNAc-T activity determined in samples isolated from healthy non afflicted individuals, a level of Core 2 GlcNAc-T higher than that in samples isolated from healthy non afflicted individuals being indicative that the subject is afflicted with Multiple Sclerosis.

13. A method of diagnosing Multiple Sclerosis in a subject according to Claim 12 wherein a level of Core 2 GlcNAc-T activity associated with samples isolated from the subject that is least 2 times higher than that in samples isolated from healthy non afflicted individuals is indicative of Multiple Sclerosis in the subject.

14. A method of diagnosing Multiple Sclerosis in a subject according to Claim 12 wherein the measurement of Core 2 GlcNAc-T is carried out on blood samples or biopsy samples.

15. A method of diagnosing Multiple Sclerosis in a subject according to Claim 12 wherein the measurement of Core 2 GlcNAc-T is carried out on isolated blood cell preparations containing leukocytes.

16. A method of diagnosing Multiple Sclerosis in a subject according to Claim 15 wherein the measurement of Core 2 GlcNAc-T is carried out on isolated blood cell preparations containing leukocytes substantially free of red blood cells.

17. A method of diagnosing Multiple Sclerosis in a subject according to any one of claims 12 to 16 wherein Multiple Sclerosis is selected from relapsing/remitting, secondary progressive, progressive relapsing, primary progressive benign, malignant, chronic/progressive and transitional/progressive Multiple Sclerosis.

18. A method of determining the utility of a substance for use in the treatment of Multiple Sclerosis comprising measuring the ability of the substance to inhibit the activity of Core 2 GlcNAc-T.

19. A method according to Claim 18 wherein the ability of the substance to inhibit the activity of Core 2 GlcNAc-T is measured by comparing the level of Core 2 GlcNAc-T activity obtained in an assay in which a test substance is incorporated to the level of Core 2 GlcNAc-T activity in the assay with no test substance.

20. A method according to either one of Claims 18 or 19 wherein the ability of the substance to inhibit the activity of Core 2 GlcNAc-T is measured by a method comprising (a) contacting a source of active Core 2 GlcNAc-T enzyme with an acceptor and a sugar donor for a Core 2 GlcNAc-T in the presence of a test substance; (b) measuring the amount of sugar donor transferred to the acceptor, and (c) carrying out steps (a) and (b) in the absence of the test substance to determine whether the substance inhibits the transfer of the sugar donor to the acceptor by the Core 2 GlcNAc-T.

21. A method according to any one of Claims 18 to 20 wherein Multiple Sclerosis is selected from relapsing/remitting, secondary progressive, progressive relapsing, primary progressive benign, malignant, chronic/progressive and transitional/progressive Multiple Sclerosis.

22. The use of a compound capable of reducing the activity of Core 2 GlcNAc-T in the manufacture of a medicament for the treatment of Multiple Sclerosis.

23. The use according to Claim22 wherein the compound is an inhibitor of Core 2 GlcNAc-T.

24. The use according to either of Claims 22 or 23 in which the inhibitor of Core 2 GlcNAc-T activity is selected from the group consisting of steroidal glycosides, analogues of Uridine Diphosphate-N-Acetylglucosamine, analogues of Uridine Diphosphate, ßGal(1→3)α(6-deoxy)GalNAcα-Bn and those compounds obtainable by UV activation of a compounds selected from the group consisting of Galß1-3GalNAcα-pnp, Galß1→3GalNAcα-onp, GalNAcα-pnp, GalNAcß-pnp,
GlcNAcß-pnp, Galß-pnp, GlcNAcß1→3GalNAcα-pnp, L-Fucα1→2Galß-pnp, GlcNAcα-pnp, Galß1→6GlcNAcß-pnp and Galß1→GlcNAcß-pnp.

25. The use according to either one of Claims 23 or 24 in which the inhibitor of Core 2 GlcNAc-T activity is a steroidal glycoside.

26. The use according to any one of Claims 22 to 25 in which the inhibitor of Core 2 GlcNAc-T activity is selected from the group consisting of Trigoneoside IVa, Glycoside F, Compound 3, Pardarinoside C, Shatavarin I, Shatavarin IV, Deltonin, Balanitin VI, solasodine 3-O-α-L-rhamnopyranosyl-(1→2)-O-[ß-D-glucopyranosyl-(1→4)]-ß-D-glucopyranoside, Solanidine 3-O-α-L-rhamnopyranosyl-(1→2)-O-[ß-D-glucopyranosyl-(1→4)]-ß-D-glucopyranoside.

27. The use according to any one of Claims 23 to 26 in which the inhibitor of Core 2 GlcNAc-T activity has been incorporated into a food or beverage product

28. The use according to Claim 27 wherein the compound capable of reducing the activity of Core 2 GlcNAc-T is an inhibitor of protein kinase Cß.

29. The use according to Claim 28 wherein the compound capable of reducing the activity of Core 2 GlcNAc-T is an inhibitor of protein kinase Cß2.

30. The use according to Claim 29 wherein the compound is selected from the group consisting of 3,4-di-indoyl-pyrrol-2,5-dione derivatives.

31. The use according to Claim 29 wherein the compound is Ruboxistaurin.

32. The use according to any one of claims 22 to 31 wherein Multiple Sclerosis is selected from relapsing/remitting, secondary progressive, progressive relapsing, primary progressive benign, malignant, chronic/progressive and transitional/progressive Multiple Sclerosis.

## Patentansprüche

1. Verbindung, die in der Lage ist, die Aktivität von Core 2 GlcNAc-T zu reduzieren, zur Verwendung bei der Behandlung von multipler Sklerose.

2. Verbindung zur Verwendung nach Anspruch 1, wobei die Verbindung, die in der Lage ist, die Aktivität von Core 2 GIcNAc-T zu reduzieren, ein Inhibitor von Core 2 GlcNAc-T-Enzymaktivität ist.

3. Verbindung zur Verwendung entweder nach Anspruch 1 oder 2, wobei der Inhibitor von Core 2 GlcNAc-T-Aktivität aus der Gruppe ausgewählt ist, bestehend aus Steroidglycosiden, Analogen von Uridindiphosphat-N-acetylglucosamin, Analogen von Uridindiphosphat, βGal(1→3)α(6-deoxy)GalNAcα-Bn und denjenigen Verbindungen, die durch UV-Aktivierung einer Verbindung zugänglich sind, die aus der Gruppe ausgewählt ist, bestehend aus Galß1→3GalNAcα-pnp, Galβ1→GalNAcα-onp, GalNAcα-pnp, GalNAcβ-pnp, GlcNAcβ-pnp, Galβ-pnp, GlcNAcß1→3GalNAcα-pnp, L-Fucα1→2Galβ-pnp, GlcNAcα-pnp, Galβ1→6GlcNAcβ-pnp und Galβ1→3GlcNAcβ-pnp.

4. Verbindung zur Verwendung nach einem der Ansprüche 2 oder 3, wobei der Inhibitor von Core 2 GlcNAc-T-Aktivität ein Steroidglycosid ist.

5. Verbindung zur Verwendung nach einem der Ansprüche 2 bis 4, wobei der Inhibitor von Core 2 GlcNAc-T-Aktivität aus der Gruppe ausgewählt ist, bestehend aus Trigoneosid IVa, Glycosid F, Verbindung 3, Pardarinosid C, Shatavarin I, Shatavarin IV, Deltonin, Balanitin VI, Solasodin-3-O-α-L-rhamnopyranosyl-(1→2)-O[β-D-glucopyranosyl-(1→4)]-β-D-glucopyranosid, Solanidin-3-O-α-L-rhamnopyranosyl-(1→2)-O[β-D-glucopyranosyl-(1→4)]-β-D-glucopyranosid.

6. Verbindung zur Verwendung nach einem der Ansprüche 2 bis 5, wobei der Inhibitor von Core 2 GlcNAc-T-Aktivität in ein Lebensmittel- oder Getränkeprodukt eingearbeitet wurde.

7. Verbindung zur Verwendung nach Anspruch 1, wobei die Verbindung, die in der Lage ist, die Core 2 GlcNAc-T-Aktivität zu reduzieren, ein Inhibitor der Proteinkinase Cβ ist.

8. Verbindung zur Verwendung nach Anspruch 7, wobei die Verbindung, die in der Lage ist, die Aktivität von Core 2 GlcNAc-T zu reduzieren, ein Inhibitor der Proteinkinase Cβ2 ist.

9. Verbindung zur Verwendung nach Anspruch 8, wobei die Verbindung aus der Gruppe bestehend aus 3,4-Di-indoyl-pyrrol-2,5-dion-Derivaten ausgewählt ist.

10. Verbindung zur Verwendung nach Anspruch 8, wobei die Verbindung Ruboxistaurin ist.

11. Verbindung zur Verwendung nach einem der Ansprüche 1 bis 10, wobei die Multiple Sklerose aus relapsierender/remittierender, sekundär progressiver, progressiv relapsierender, primär progressiver, gutartiger, bösartiger, chronischer/progressiver und transitionaler/progressiver Multipler Sklerose ausgewählt ist.

12. Verfahren zur Diagnose von Multipler Sklerose in einem Individuum, umfassend das Vergleichen des Niveaus von Core 2 GlcNAc-T-Aktivität, die mit aus dem Individuum isolierten Proben assoziiert ist, mit dem Niveau von Core 2 GlcNAc-T-Aktivität, die in Proben bestimmt wurde, die aus gesunden nicht befallenen Individuen isoliert wurden, wobei ein Niveau von Core 2 GlcNAc-T, das höher ist als in Proben, die aus gesunden nicht befallenen Individuen isoliert wurden, ein Hinweis darauf ist, dass das Individuum von Multipler Sklerose befallen ist.

13. Verfahren zur Diagnose von Multipler Sklerose in einem Individuum nach Anspruch 12, wobei ein Niveau von Core 2 GlcNAc-T-Aktivität, die mit aus dem Individuum isolierten Proben assoziiert ist, das mindestens zwei Mal höher ist als dasjenige in Proben, die aus gesunden nicht befallenen Individuen isoliert wurden, ein Hinweis auf Multiple Sklerose in dem Individuum ist.

14. Verfahren zur Diagnose von Multipler Sklerose in einem Individuum nach Anspruch 12, wobei die Messung von Core 2 GIcNAc-T an Blutproben oder Biopsieproben durchgeführt wird.

15. Verfahren zur Diagnose von Multipler Sklerose in einem Individuum nach Anspruch 12, wobei die Messung von Core 2 GlcNAc-T an isolierten Blutzellpräparationen, die Leukozyten enthalten, durchgeführt wird.

16. Verfahren zur Diagnose von Multipler Sklerose in einem Individuum nach Anspruch 15, wobei die Messung von Core 2 GlcNAc-T an Blutzellpräparationen, die Leukozyten enthalten, die im Wesentlichen frei von roten Blutzellen sind, durchgeführt wird.

17. Verfahren zur Diagnose von Multipler Sklerose in einem Individuum nach einem der Ansprüche 12 bis 16, wobei die Multiple Sklerose aus relapsierender/remittierender, sekundär progressiver, progressiv relapsierender, primär progressiver, gutartiger, bösartiger, chronischer/progressiver und transitionaler/progressiver Multipler Sklerose ausgewählt ist.

18. Verfahren zur Bestimmung der Brauchbarkeit einer Substanz zur Verwendung bei der Behandlung von Multipler Sklerose umfassend das Messen der Fähigkeit der Substanz zur Hemmung der Aktivität von Core 2 GlcNAc-T.

19. Verfahren nach Anspruch 18, wobei die Fähigkeit der Substanz zur Hemmung der Aktivität von Core 2 GlcNAc-T durch Vergleichen des Niveaus von Core 2 GlcNAc-T-Aktivität, die in einem Assay, wobei eine Testsubstanz dem Niveau von Core 2 GlcNAc-T-Aktivität einverleibt wurde, erhalten wurde, mit dem Niveau von Core 2 GlcNAc-T-Aktivität in dem Assay ohne Testsubstanz gemessen wird.

20. Verfahren nach einem der Ansprüche 18 oder 19, wobei die Fähigkeit der Substanz zur Hemmung der Aktivität von Core 2 GlcNAc-T durch ein Verfahren gemessen wird, umfassend (a) Kontaktieren einer Quelle von aktivem Core 2 GlcNAc-T-Enzym mit einem Akzeptor und einem Zucker-Donor für ein Core 2 GlcNAc-T in Gegenwart einer Testsubstanz; (b) Messen der auf den Akzeptor übertragenen Menge an Zucker-Donor, und (c) Durchführen der Schritte (a) und (b) in Abwesenheit der Testsubstanz, um zu bestimmen, ob die Substanz die Übertragung des Zuckerdonors auf den Akzeptor durch das Core 2 GlcNAc-T hemmt.

21. Verfahren nach einem der Ansprüche 18 bis 20, wobei die Multiple Sklerose aus relapsierender/remittierender, sekundär progressiver, progressiv relapsierender, primär progressiver, gutartiger, bösartiger, chronischer/progressiver und transitionaler/progressiver Multipler Sklerose ausgewählt ist.

22. Verwendung einer Verbindung, die in der Lage ist, die Aktivität von Core 2 GIcNAc-T zu reduzieren, bei der Herstellung eines Medikaments zur Behandlung von Multipler Sklerose.

23. Verwendung nach Anspruch 22, wobei die Verbindung ein Inhibitor von Core 2 GIcNAc-T ist.

24. Verwendung nach einem der Ansprüche 22 oder 23, wobei der Inhibitor von Core 2 GlcNAc-T-Aktivität aus der Gruppe ausgewählt ist, bestehend aus Steroidglycosiden, Analogen von Uridindiphosphat-N-acetylglucosamin, Analogen von Uridindiphosphat, βGal(1→3)α(6-deoxy)GalNAcα-Bn und denjenigen Verbindungen, die durch UV-Aktivierung einer Verbindung zugänglich sind, die aus der Gruppe ausgewählt ist, bestehend aus Galβ1→2GalNAcα-pnp, Galβ1→3GalNAcα-pnp, GalNAcα-pnp, GalNAcβ-pnp, GlcNAcβ-pnp, Galβ-pnp, GlcNAcβ1→3GalNAcα-pnp, L-Fuca1-2Galβ-pnp, GlcNAcα-pnp, Galβ1→6GlcNAcβ-pnp und Galβ1→3GlcNAcβ-pnp.

25. Verwendung nach einem der Ansprüche 23 oder 24, wobei der Inhibitor von Core 2-Aktivität ein Steroidglycosid ist.

26. Verwendung nach einem der Ansprüche 22 bis 25, wobei der Inhibitor von Core 2 GlcNAc-T-Aktivität aus der Gruppe ausgewählt ist bestehend aus Trigoneosid IVa, Glycosid F, Verbindung 3, Pardarinosid C, Shatavarin I, Shatavarin IV, Deltonin, Balanitin VI, Solasodin-3-O-α-L-rhamnopyranosyl-(1→2)-O[β-D-glucopyranosyl-(1→4)]-β-D-glucopyranoside, Solanidin-3-O-α-L-rhamnopyranosyl-(1→2)-O[β-D-glucopyranosyl-(1→4)]-β-D-glucopyranosid.

27. Verwendung nach einem der Ansprüche 23 bis 26, wobei der Inhibitor von Core 2 GlcNAc-T-Aktivität in ein Lebensmittel- oder Getränkeprodukt eingearbeitet ist.

28. Verwendung nach Anspruch 27, wobei die Verbindung, die in der Lage ist, die Aktivität von Core 2 GlcNAc-T zu reduzieren, ein Inhibitor von Proteinkinase Cβ ist.

29. Verwendung nach Anspruch 28, wobei die Verbindung, die in der Lage ist, die Aktivität von Core 2 GIcNAc-T zu reduzieren, ein Inhibitor von Proteinkinase Cβ2 ist.

30. Verwendung nach Anspruch 29, wobei die Verbindung aus der Gruppe bestehend aus 3,4-Di-indoyl-pyrrol-2,5-dion-Derivaten ausgewählt ist.

31. Verwendung nach Anspruch 29, wobei die Verbindung Ruboxistaurin ist.

32. Verwendung nach einem der Ansprüche 22 bis 31, wobei die Multiple Sklerose aus relapsierender/remittierender, sekundär progressiver, progressiv relapsierender, primär progressiver, gutartiger, bösartiger, chronischer/progressiver und transitionaler/progressiver Multipler Sklerose ausgewählt ist.

## Revendications

1. Composé capable de réduire l'activité de Core 2 GlcNAc-T destiné à être utilisé dans le traitement de la sclérose en plaques.

2. Composé destiné à être utilisé selon la revendication 1 où le composé capable de réduire l'activité de Core 2 GlcNAc-T est un inhibiteur de l'activité de l'enzyme Core 2 GlcNAc-T.

3. Composé destiné à être utilisé selon la revendication 1 ou 2 où l'inhibiteur de l'activité de Core 2 GlcNAc-T est choisi dans le groupe consistant en les glycosides stéroïdiens, les analogues d'uridine diphosphate-N-acétylglucosamine, les analogues d'uridine diphosphate, ßGal(1→3)α(6-désoxy)GalNAcα-Bn et les composés pouvant être obtenus par activation UV d'un composé choisi dans le groupe consistant en Galß1→3GalNAcα-pnp, Galß1→3GalNAcα-onp, GalNAcα-pnp, GalNAcß-pnp, GlcNAcß-pnp, Galß-pnp, GlcNAcß1→3GalNAcα-pnp, L-Fucα1→3Galß-pnp, GlcNAcα-pnp, Galß1→6GlcNAcß-pnp et Galß1→3GlcNAcß-pnp.

4. Composé destiné à être utilisé selon la revendication 2 ou 3 où l'inhibiteur de l'activité de Core 2 GlcNAc-T est un glycoside stéroïdien.

5. Composé destiné à être utilisé selon l'une quelconque des revendications 2 à 4 où l'inhibiteur de l'activité de Core 2 GlcNAc-T est choisi dans le groupe consistant en le trigonéoside IVa, le glycoside F, le composé 3, le pardarinoside C, la shatavarine I, la shatavarine IV, la deltonine, la balanitine VI, la solasodine 3-O-α-L-rhamnopyranosyl-(1→2)-O-[ß-D-glucopyranosyl-(1→4)]-ß-D-glucopyranoside, la solanidine 3-O-α-L-rhamnopyranosyl-(1→2)-O-[ß-D-glucopyranosyl-(1→4)]-ß-D-glucopyranoside.

6. Composé destiné à être utilisé selon l'une quelconque des revendications 2 à 5 où l'inhibiteur de l'activité de Core 2 GlcNAc-T a été incorporé dans un produit de type aliment ou boisson.

7. Composé destiné à être utilisé selon la revendication 1 où le composé capable de réduire l'activité de Core 2 GlcNAc-T est un inhibiteur de protéine kinase Cß.

8. Composé destiné à être utilisé selon la revendication 7 où le composé capable de réduire l'activité de Core 2 GIcNAc-T est un inhibiteur de protéine kinase Cß2.

9. Composé destiné à être utilisé selon la revendication 8 où le composé est choisi dans le groupe consistant en les dérivés de 3,4-di-indoyl-pyrrole-2,5-dione.

10. Composé destiné à être utilisé selon la revendication 8 où le composé est la ruboxistaurine.

11. Composé destiné à être utilisé selon l'une quelconque des revendications 1 à 10 où la sclérose en plaques est choisie parmi la sclérose en plaques récidivante/rémittente, progressive secondaire, récidivante progressive, bénigne progressive primaire, maligne, chronique/progressive et transitionnelle/progressive.

12. Procédé de diagnostic de la sclérose en plaques chez un sujet comprenant la comparaison du niveau d'activité de Core 2 GlcNAc-T associée avec des échantillons isolés sur le sujet avec le niveau d'activité de Core 2 GIcNAc-T déterminé dans des échantillons isolés sur des individus non atteints sains, un niveau de Core 2 GIcNAc-T supérieur à celui dans des échantillons isolés sur des individus non atteints sains indiquant que le sujet est atteint de sclérose en plaques.

13. Procédé de diagnostic de la sclérose en plaques chez un sujet selon la revendication 12 où un niveau d'activité de Core 2 GlcNAc-T associé avec des échantillons isolés sur le sujet qui est au moins 2 fois plus élevé que celui dans des échantillons isolés sur des individus non atteints sains indique la sclérose en plaques chez le sujet.

14. Procédé de diagnostic de la sclérose en plaques chez un sujet selon la revendication 12 où la mesure de Core 2 GlcNAc-T est réalisée sur des échantillons sanguins ou des échantillons de biopsie.

15. Procédé de diagnostic de la sclérose en plaques chez un sujet selon la revendication 12 où la mesure de Core 2 GlcNAc-T est réalisée sur des préparations de cellules sanguines isolées contenant des leucocytes.

16. Procédé de diagnostic de la sclérose en plaques chez un sujet selon la revendication 15 où la mesure de Core 2 GIcNAc-T est réalisée sur des préparations de cellules sanguines isolées contenant des leucocytes sensiblement dépourvues de globules rouges.

17. Procédé de diagnostic de la sclérose en plaques chez un sujet selon l'une quelconque des revendications 12 à 16 où la sclérose en plaques est choisie parmi la sclérose en plaques récidivante/rémittente, progressive secondaire, récidivante progressive, bénigne progressive primaire, maligne, chronique/progressive et transitionnelle/progressive.

18. Procédé de détermination de l'utilité d'une substance destinée à être utilisée dans le traitement de la sclérose en plaques comprenant la mesure de l'aptitude de la substance à inhiber l'activité de Core 2 GlcNAc-T.

19. Procédé selon la revendication 18 où l'aptitude de la substance à inhiber l'activité de Core 2 GIcNAc-T est mesurée en comparant le niveau d'activité de Core 2 GIcNAc-T obtenu dans un essai où une substance test est incorporée au niveau d'activité de Core 2 GIcNAc-T dans l'essai sans substance test.

20. Procédé selon la revendication 18 ou 19 où l'aptitude de la substance à inhiber l'activité de Core 2 GlcNAc-T est mesurée par un procédé comprenant (a) la mise en contact d'une source d'enzyme Core 2 GlcNAc-T active avec un accepteur et un donneur de sucre pour une Core 2 GIcNAc-T en présence d'une substance test ; (b) la mesure de la quantité de donneur de sucre transférée à l'accepteur, et (c) la mise en oeuvre des étapes (a) et (b) en l'absence de la substance test pour déterminer si la substance inhibe le transfert du donneur de sucre à l'accepteur par la Core 2 GlcNAc-T.

21. Procédé selon l'une quelconque des revendications 18 à 20 où la sclérose en plaques est choisie parmi la sclérose en plaques récidivante/rémittente, progressive secondaire, récidivante progressive, bénigne progressive primaire, maligne, chronique/progressive et transitionnelle/progressive.

22. Utilisation d'un composé capable de réduire l'activité de Core 2 GlcNAc-T dans la fabrication d'un médicament pour le traitement de la sclérose en plaques.

23. Utilisation selon la revendication 22 où le composé est un inhibiteur de Core 2 GlcNAc-T.

24. Utilisation selon la revendication 22 ou 23 où l'inhibiteur de l'activité de Core 2 GIcNAc-T est choisi dans le groupe consistant en les glycosides stéroïdiens, les analogues d'uridine diphosphate-N-acétylglucosamine, les analogues d'uridine diphosphate, ßGal(1→3)α(6-désoxy)GalNAcα-Bn et les composés pouvant être obtenus par activation UV d'un composé choisi dans le groupe consistant en Galß1→3GalNAcα-pnp, Galß1→3GalNAcα-onp, GalNAcα-pnp, GalNAcß-pnp, GlcNAcß-pnp, Galß-pnp, GlcNAcß1→3GalNAcα-pnp, L-Fucα1→3Galß-pnp, GlcNAcα-pnp, Galß1→6GlcNAcß-pnp et Galß1→3GlcNAcß-pnp.

25. Utilisation selon la revendication 23 ou 24 où l'inhibiteur de l'activité de Core 2 GIcNAc-T est un glycoside stéroïdien.

26. Utilisation selon l'une quelconque des revendications 22 à 25 où l'inhibiteur de l'activité de Core 2 GIcNAc-T est choisi dans le groupe consistant en le trigonéoside IVa, le glycoside F, le composé 3, le pardarinoside C, la shatavarine I, la shatavarine IV, la deltonine, la balanitine VI, la solasodine 3-O-α-L-rhamnopyranosyl-(1→2)-O-[ß-D-glucopyranosyl-(1→4)]-ß-D-glucopyranoside, la solanidine 3-O-α-L-rhamnopyranosyl-(1→2)-O-[ß-D-glucopyranosyl-(1→4)]-ß-D-glucopyranoside.

27. Utilisation selon l'une quelconque des revendications 23 à 26 où l'inhibiteur de l'activité de Core 2 GlcNAc-T a été incorporé dans un produit de type aliment ou boisson.

28. Utilisation selon la revendication 27 où le composé capable de réduire l'activité de Core 2 GIcNAc-T est un inhibiteur de protéine kinase Cß.

29. Utilisation selon la revendication 28 où le composé capable de réduire l'activité de Core 2 GlcNAc-T est un inhibiteur de protéine kinase Cß2.

30. Utilisation selon la revendication 29 où le composé est choisi dans le groupe consistant en les dérivés de 3,4-di-indoyl-pyrrole-2,5-dione.

31. Utilisation selon la revendication 29 où le composé est la ruboxistaurine.

32. Utilisation selon l'une quelconque des revendications 22 à 31 où la sclérose en plaques est choisie parmi la sclérose en plaques récidivante/rémittente, progressive secondaire, récidivante progressive, bénigne progressive primaire, maligne, chronique/progressive et transitionnelle/progressive.
